# EUROPEAN PATENT APPLICATION

(11) **EP 3 733 011 A1**
(43) Date of publication of application: **04.11.2020**
(21) Application number: 18904309.4
(22) Date of filing: 24.10.2018
(51) Int. Cl.: A43B 3/00, A61B 5/103, G08B 21/18

(54) **WORKING SHOE COMPRISING SENSOR**

(30) Priority: 02.02.2018 KR 20180013637
(71) Applicant: SAMDUK TONGSANG CO., LTD., Gangseo-gu Busan 46728 (KR)
(72) Inventor: JUN, Sung Pyo, Busan 46232 (KR); YU, Won Ho, Busan 46726 (KR); SHIM, Jae Ryun, Busan 47150 (KR); JUNG, In Bae, Busan 49416 (KR)
(74) Representative: Isarpatent
(86) International application number: PCT/KR2018/012655
(87) International publication number: WO 2019/151612

(57) **Abstract**

One aspect of the present invention provides work shoes each including an outsole, a midsole, an inner board, and a mid-insole from the ground up, wherein a thickness of the mid-insole is twice or more a thickness of the midsole, the mid-insole includes a substrate including a plurality of first sensors, an electronic module, and a wiring configured to connect the first sensors and the electronic module, and a body part in which a groove, into which the electronic module downwardly protruding from the substrate is inserted, is formed, the electronic module includes a communication module and a battery, the communication module communicates with an external device, and the external device analyzes information received from work shoes and then notifies a worker and a manager of the information .

## Description

### [Technical Field]

The present invention relates to work shoes which protect the feet of a worker, and more specifically, to work shoes each including a sensor which senses a body condition of a worker through the feet and a communication module which transmits sensed information to an external device.

### [Background Art]

A conventional work shoe is formed in the ascending order of an outsole, a midsole, an inner board, an insole, and a shoe insert from the ground up.

When an excessive load is applied to feet, a response of a human body to the load occurs, and when the load repeatedly and continuously acts, the possibility of injury can increase. To prevent this, it is necessary to embed a sensor in the work shoe to measure and evaluate the load. Further, a battery which operates the sensor and a communication module which transmits a signal from the sensor to an external device are required. In order to apply the configuration to the work shoe, first, a worker should be able to work by conveniently wearing the shoes, and also should meet strict standards (see protective equipment performance test regulations) on the outsole, inner board, and the like of the work shoe.

Korean Utility Model Registration No. 20-0229752 discloses a load alarm device for work shoes to safely protect workers from occupational diseases and industrial accidents such as personal injuries or back pain during laborious transport work at industrial sites. However, a sensing means which is one component of the alarm device, can be easily damaged by a pointed object such as a nail or the like under the inner board, and the inner board can interfere with the detection of a signal by a worker. Further, when failures of the sensing means and the alarm device occur, there is a hassle in that the outsole should be opened or the insole and the inner board should be torn off to repair the sensing means and the alarm device.

Korean Laid-Open Patent No. 10-2012-0135934 discloses artificial intelligence shoes which is worn by a user to be capable of calculating a movement amount through a signal of a sensor due to movement or checking the movement amount in real time by transmitting and receiving the signal to/from a portable device, and is chargeable and includes a movement amount check training system of the user through connection to a computer by adopting a universal serial bus (USB) type sensor therein. However, charging through a USB is vulnerable to moisture, and can only be used indoors with connection to the computer. Further, the artificial intelligence shoes can be used only in the laboratory or indoors to sense a body condition of the user, and cannot be used in various fields.
(Prior art document 1) Korean Utility Model Registration No. 20-0229752
(Prior art document 2) Korean Laid-Open Patent No. 10-2012-0135934

### [Disclosure]

### [Technical Problem]

The present invention is directed to providing work shoes each having at least one built-in sensor, without compromising worker protection and convenience of the work shoes.

Further, the present invention is directed to providing work shoes in which built-in sensors are smoothly operated depending on the operation of a worker without interference from other components.

In addition, the present invention is directed to providing work shoes capable of guaranteeing complete waterproofing of components and protected from a risk of damage due to a nail or the like.

In addition, the present invention is directed to providing work shoes which are easily mended or replaceable when components fail.

In addition, the present invention is directed to providing work shoes which are linkable with other wearable devices.

### [Technical Solution]

One aspect of the present invention provides work shoes each including an outsole, a midsole, an inner board, and a mid-insole from the ground up, wherein a thickness of the mid-insole is twice or more a thickness of the midsole, the mid-insole includes a substrate including a plurality of first sensors, an electronic module, and a wiring configured to connect the first sensors and the electronic module, and a body part in which a groove, into which the electronic module downwardly protruding from the substrate is inserted, is formed, the electronic module includes a communication module and a battery, the communication module communicates with an external device, and the external device analyzes information received from work shoes and then notifies a worker and a manager of the information.

In one embodiment, the first sensors may be located at toe regions, a ball region, and a heel region of the substrate.

In one embodiment, each of the first sensors may be one selected from the group consisting of a pressure sensor, a temperature sensor, a humidity sensor, a three-axis acceleration sensor, a three-axis gyro sensor, a three-axis geomagnetic sensor, a global positioning system (GPS) sensor, and a combination of two or more thereof.

In one embodiment, the substrate may be embedded in the mid-insole.

In one embodiment, the communication module may communicate with a second sensor attached to the body of a worker.

In one embodiment, the second sensor may be attached to a joint region of the worker, a plurality of bone regions connected to the joint region, or a combination thereof.

In one embodiment, the work shoes may each further include an antibacterial layer on the mid-insole.

In one embodiment, the electronic module may be located at an arch region of the substrate, and the wiring may extend from the first sensor to form a port and an interface at an inner arch region of the substrate and may be connected to the electronic module.

### [Advantageous Effects]

In each of work shoes according to one aspect of the present invention, at least one sensor can be mounted on the work shoes without spoiling the protection and convenience of a worker, and the mounted sensor can be smoothly operated without interference from other components depending on an operation of the worker.

Further, the work shoes can guarantee complete waterproofing of the mounted components, can be protected from a risk of damage due to a nail or the like, can be easily mended or replaced when the component fails, can be linked with other wearable device, and can provide an antibacterial layer to prevent bacterial contamination of feet.

In addition, in the work shoe, a structure of a wiring included in a substrate can fundamentally prevent disconnection of the wiring due to load accumulation and errors and malfunctions of sensors and an electronic module caused by the above.

### [Description of Drawings]

FIG. 1 is a perspective view of a substrate according to one embodiment of the present invention.
FIG. 2 is a perspective view of a mid-insole in which the substrate, a battery, and a communication module of the present invention are embedded.
FIG. 3 is a cross-sectional view of line A-A of the mid-insole in FIG. 2.
FIG. 4 is a cross-sectional view of a sole in which the mid-insole in FIG. 2 is installed and a wireless charger.
FIG. 5 is a view illustrating a driving principle of first and second sensors according to one embodiment of the present invention.
FIG. 6 is an image of measurement of an average foot pressure.
FIG. 7 is a plan view of the substrate according to one embodiment of the present invention.

### [Modes of the Invention]

Hereinafter, the present invention will be described with reference to the accompanying drawings. However, present invention may be implemented in various forms and is not limited to the following embodiments. Further, parts not related to the description are omitted to clearly describe the present invention, and similar reference symbols are used for similar parts through the specification.

In the specification, a case in which a certain part is mentioned as being "connected" to another part includes not only a case in which the part is "directly connected" to the other part, but also a case in which the part is "indirectly connected" to the other part with another member therebetween. Further, a case in which the certain part is mentioned as "including" a certain component refers to a case in which another component may be further provided unless otherwise defined, not a case in which another component is excluded.

Hereinafter, the embodiments of the present invention will be described in detail with reference to the accompanying drawings

One aspect of the present invention provides work shoes each including an outsole, a midsole, an inner board, and a mid-insole from the ground up, wherein a thickness of the mid-insole is twice or more a thickness of the midsole, the mid-insole includes a substrate including a plurality of first sensors, an electronic module, and a wiring configured to connect the first sensors and the electronic module, and a body part in which a groove, into which the electronic module downwardly protruding from the substrate is inserted, is formed, the electronic module includes a communication module and a battery, the communication module communicates with an external device, and the external device analyzes information received from work shoes and then notifies a worker and a manager of the information .

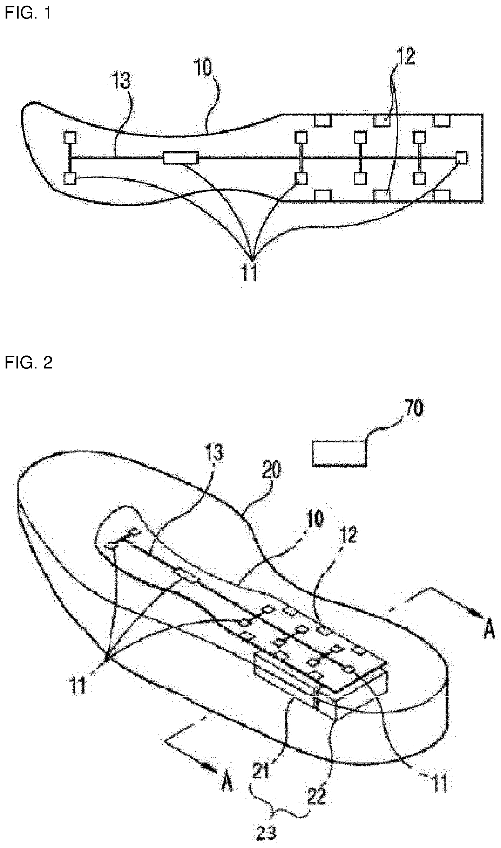

FIG. 1 is a perspective view of a substrate according to one embodiment of the present invention. Referring to FIG. 1, the substrate 10 may include a plurality of first sensors 11 which sense a state of a worker wearing work shoes, a plurality of contact points 12 which link a battery 21 and a communication module 22, and a wiring 13 which links the first sensors 11 and the contact points 12.

The substrate 10 is a thin flexible synthetic resin plate, and may be formed of a material which does not affect the cushioning of the feet of the worker at all. The first sensor 10 may be, for example, one selected from the group consisting of a pressure sensor, a temperature sensor, a humidity sensor, a three-axis acceleration sensor, a three-axis gyro sensor, a three-axis geomagnetic sensor, a global positioning system (GPS) sensor, and a combination of two or more thereof, and preferably, may be the pressure sensor but is not limited thereto.

Since pressure distribution is measured by the pressure sensors, a load or impact transmitted to each part of the human body during the activity of the worker may be precisely measured, analyzed, and evaluated, and an injury to the worker may also be prevented, treated, and diagnosed.

Further, an arbitrary portion of the substrate may further include the temperature sensor and the humidity sensor to measure a state of feet of the worker and a working environment. The temperature sensor may prevent an operator who works in an extreme environment, for example, an extremely low temperature region and an extremely high temperature region, from exposure to danger associated with extreme temperature. For example, when the temperature sensor senses that a temperature in the area is 30°C or more, the worker may stop working and move to a safe place.

Further, one or more of the three-axis acceleration sensor, the three-axis gyro sensor, the three-axis geomagnetic sensor, the GPS sensor may be installed in the substrate as an additional sensor. The three-axis acceleration sensor may measure inclination, acceleration, vibrations, and an impact when the worker stops. The three-axis gyro sensor may measure a change amount when a position of the worker is changed. Further, the three-axis geomagnetic sensor may grasp an accurate orientation of the worker through a magnetic field. A walking distance for a task of the worker may be calculated by the GPS sensor, and a position of the worker may be quickly grasped in the event of an accident.

FIG. 2 is a perspective view of a mid-insole in which the substrate, the battery, and the communication module of the present invention are embedded. Referring to FIG. 2, the mid-insole 20 may include the substrate 10 including the wiring 13 which connects the first sensors 11 and an electronic module 23 and a body part in which a groove, into which the electronic module 23 downwardly protruding from the substrate 10 is inserted, is formed, and the electronic module 23 may include the communication module 22 and the battery 21.

The substrate 10 may be embedded in the mid-insole 20 or attached and coupled to a surface of the mid-insole 20. For example, the midsole and the insole may be made as one member to embed the substrate 10, the battery 21, and the communication module 22 therein.

A thickness of the mid-insole is twice or more a thickness of the midsole. Although a battery is embedded in a midsole under a conventional inner board and thus there is the above-described problem, in the work shoe according to one embodiment of the present invention, the battery is embedded on the inner board.

The substrate 10 may have a shape corresponding to a shape of the foot of the worker, and the first sensors 11 may mounted according to the shape of the foot of the worker. The substrate 10 may be substantially located on the mid-insole 20 to sense a load on the foot.

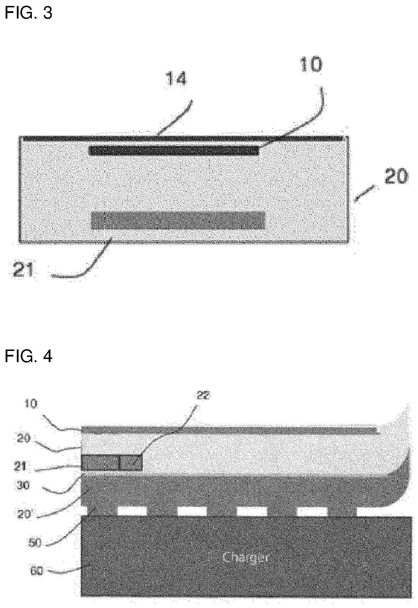

FIG. 3 is a cross-sectional view of line A-A of the mid-insole in FIG. 2. Referring to FIG. 3, the battery 21 and the communication module 22 may be located at a lower portion of the mid-insole 20 in a manner of not affecting the cushioning by the mid-insole 20. The substrate 10 may be located at an upper portion close to the foot of the worker to ensure a smooth operation of the first sensors 11.

Further, an antibacterial layer 14 may be coated on the mid-insole 20. The antibacterial layer 14 may be one selected from the group consisting of silver, zinc, copper, tin, platinum, barium, magnesium, germanium, titanium, calcium, and a combination of two or more thereof, but is not limited thereto.

Since the substrate 10, the battery 21, and the communication module 22 are all sealed in the mid-insole 20 and may be embedded in a waterproof state, corrosion and damage to components due to moisture may be prevented. The components may be understood as a concept including all components used in the work shoes as well as the first sensors, the battery, the communication module, and the wiring which connects the above.

FIG. 4 is a cross-sectional view of a sole in which the mid-insole in FIG. 2 is installed and a wireless charger. Referring to FIGS. 2 to 4, the electronic module 23 including the battery 21 and the communication module 22 may be located at an inner arch portion, which is the thickest portion of the mid-sole 20, that is, near the inner board 30 opposite the sensor, to affect the cushioning of the worker's foot less.

The battery 21 supplies power to the first sensors 11 of the substrate 10 through the wiring 13, and the communication module 22 may also receive signals and data from the first sensors 11 of the substrate 10 through the wiring 13 and transmit the signals and data to an external device 70.

The external device 70 be a mobile phone terminal for a worker, a mobile phone terminal for a manager, a company management server, a hospital server, or the like.

A communication method between the communication module 22 and the external device 70 may be Bluetooth in the case of a short distance, and may be Wi-Fi, wireless communication (radio), or the Internet, but is not limited thereto.

A control method and a communication method of the first sensors 11, the communication module 22, and the external device 70 may be applied with reference to disclosures in Korean Patent Publication No. 10-2013-7024781 and Korean Patent Publication No. 10-2012-0126930.

Referring to FIG. 4, since a charge coil is embedded in the battery 21, the battery 21 may be wirelessly charged by the wireless charger 60. The sole of the work shoe may have a structure in which an outsole 50, a midsole 20' having a smaller thickness than the midsole of the conventional art, the inner board 30, and the mid-insole 20 are sequentially stacked from the bottom. The mid-insole 20 may be thicker than the outsole 50, and the outsole and the inner board may be used in the same manner as described in "Notification of Protection Performance Test Regulations" as in the conventional art.

In the work shoe, since the mid-insole 20 is located on the inner board 30, the battery 21 and the communication module 22 embedded in the mid-insole 20 has no risk of being damaged by a nail or the like.

Meanwhile, the communication module 22 may wirelessly transmit and receive data to/from a second sensor attached to the body of the worker in addition to the first sensors 11 located on the substrate 10 of the work shoe to additionally check a condition of the worker. For example, the second sensor may be attached to a joint region of the worker, a plurality of bone regions connected at the joint region, or a combination thereof. The terms "joint region" and "bone region" used in this specification may refer to the epidermis located at an upper portion of a joint or bone of a worker and the periphery thereof, and the term "attachment" may be understood as a concept including not only attachment by adhesives, pressure adhesives, or the like but also physical or mechanical coupling using a band or the like.

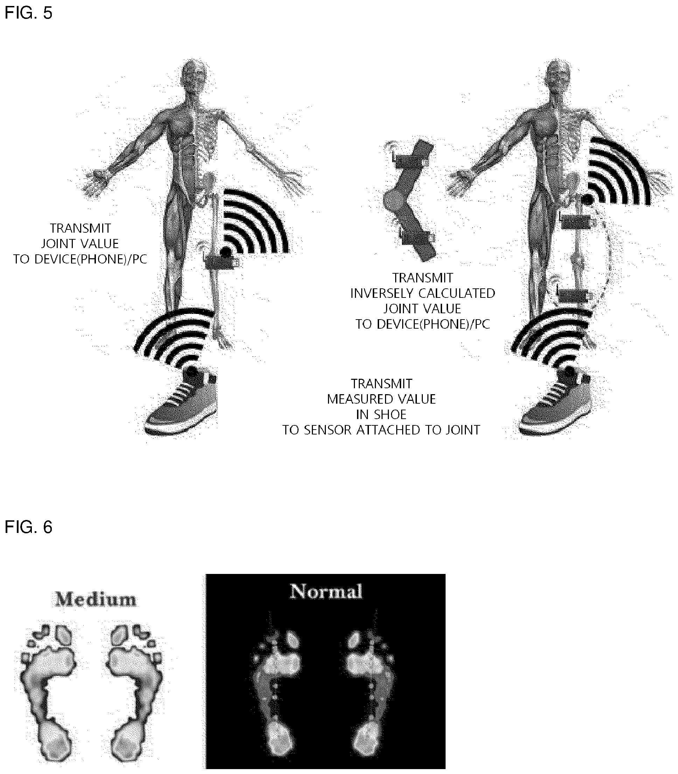

FIG. 5 is a view illustrating a driving principle of the first and second sensors according to one embodiment of the present invention. Referring to FIG. 5, when the second sensor is mounted at the joint region of the worker, for example, a knee joint, a hip joint, a spinal joint, or a cervical joint, the communication module 22 may transmit all the data through the first sensors 11 and the data through the second sensor of the joint region to the external device 70. In this case, data on the joint movement amount of the worker and a foot load is analyzed and may be used to prevent the risk of injury to the worker.

Further, the second sensor may be attached to the plurality of bone regions connected at the joint region. For example, the second sensor may be attached to a shinbone (tibia) region and a femur (thigh bone) region connected at the knee joint, that is, a calf and a thigh, respectively. The second sensor attached to the shinbone region and the femur region may track and inversely calculate a load applied to the joint by the activity of the worker and then transmit the inversely calculated load to the external device 70.

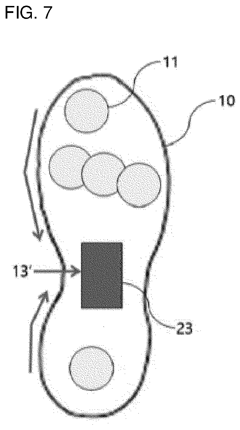

FIG. 6 is an image of measurement of an average foot pressure, and FIG. 7 is a plan view of the substrate according to one embodiment of the present invention. Referring to FIG. 6, a foot pressure applied to the shoe during the activity of the worker, that is, the load, is mainly concentrated in the toe regions, ball region, and heel region, conversely, almost no load is applied to an arch region, preferably, the inner arch region.

According to an average foot pressure distribution, like the conventional art, for example, as shown in Korean Registration Patent No. 10-1780654, when a plurality of wirings extending from the sensors and the electronic module located at the arch region are connected between the ball region and the arch region of the foot to form an interface, since the interface is located in a region where a load is large due to deformation of the work shoe, errors or malfunctions of the sensors and the electronic module may be caused when a disconnection occurs in the wiring due to load accumulation.

In this regard, referring to FIG. 7, the electronic module 23 may be located at the arch region of the substrate 10, and the wiring 13 may extend from the first sensor 11 to form a port and the interface at the inner arch region of the substrate 10 and may be connected to the electronic module 23. The structure of the wiring 13 may fundamentally prevent the disconnection of the wiring due to the load accumulation and the errors or the malfunctions of the first sensors and the electronic module due to the disconnection of the wiring.

The above description of the present invention is exemplary, and it may be understood by those skilled in the art that it is easy to modify into different concrete forms without changing the technical spirit or essential characteristic of the present invention. Accordingly, it should be understood that the above-described embodiments are completely exemplary in all respects and not limited. For example, each component described as a single type may be implemented to be distributed, and likewise, components described as being distributed may be implemented in a combined form.

The scope of the present invention is shown by the appended claims, and it should be understood that all changes or modifications derived from the meaning and scope of the claims and their equivalents are included in the scope of the present invention.

### [Reference Numerals]

10: substrate
11: sensor
12: contact point
13, 13': wirings
20: mid-insole
21: battery
22: communication module
23: electronic module
30: inner board
50: outsole
70: external device

## Claims

1. Work shoes each including an outsole, a midsole, an inner board, and a mid-insole from the ground up,
wherein a thickness of the mid-insole is twice or more a thickness of the midsole,
the mid-insole includes a substrate including a plurality of first sensors, an electronic module, and a wiring configured to connect the first sensors and the electronic module, and a body part in which a groove, into which the electronic module downwardly protruding from the substrate is inserted, is formed,
the electronic module includes a communication module and a battery,
the communication module communicates with an external device, and
the external device analyzes information received from the work shoes and then notifies a worker and a manager of the information.

2. The work shoes of claim 1, wherein the first sensors are located at toe regions, a ball region, and a heel region of the substrate.

3. The work shoes of claim 1, wherein each of the first sensors is one selected from the group consisting of a pressure sensor, a temperature sensor, a humidity sensor, a three-axis acceleration sensor, a three-axis gyro sensor, a three-axis geomagnetic sensor, a global positioning system (GPS) sensor, and a combination of two or more thereof.

4. The work shoes of claim 1, wherein the substrate is embedded in the mid-insole.

5. The work shoes of claim 1, wherein the communication module communicates with a second sensor attached to the body of a worker.

6. The work shoes of claim 5, wherein the second sensor is attached to a joint region of the worker, a plurality of bone regions connected to the joint region, or a combination of the above.

7. The work shoes of claim 1, wherein the work shoes further comprise an antibacterial layer on the mid-insole.

8. The work shoes of claim 1, wherein:
the electronic module is located at an arch region of the substrate; and
the wiring extends from the first sensor to form a port and an interface at an inner arch region of the substrate and is connected to the electronic module.
